# EUROPEAN PATENT APPLICATION

(11) **EP 1 865 076 A1**
(43) Date of publication of application: **12.12.2007**
(21) Application number: 07252244.4
(22) Date of filing: 04.06.2007
(51) Int. Cl.: C12Q 1/68

(54) **Detection of lymph node metastasis from gastric carcinoma**

(30) Priority: 05.06.2006 US 447255
(71) Applicant: Veridex, LLC, Warren, NJ 07059 (US)
(72) Inventor: Cao, Sean Wuxiong, Three Bridges, NJ 08887 (US); Green, George, Newton, NJ 07860 (US); Jones, Jennifer, Morris Plains, NJ 07950 (US); Xu, Ming, Berkeley Heights, NJ 07922 (US)
(74) Representative: Mercer, Christopher Paul

(57) **Abstract**

Methods, compositions and articles are directed to diagnosing the lymph node metastasis from gastric carcinoma, differentiating between negative lymph nodes and lymph nodes with metastasis from gastric carcinoma, testing tissue samples of for determining gastric carcinoma staging status.

## Description

### BACKGROUND OF THE INVENTION

There are about 876,000 new stomach cancer cases each year worldwide (Shibuya, Mathers et al. 2002). In the United States, the estimated new cases and deaths in 2005 for stomach cancer are 21,860 and 11,550 respectively (Jemal, Murray et al. 2005). Gastric carcinoma is the most common type (90% to 95%) of stomach cancer, which is followed in the order of incidence by lymphomas, carcinoids and mesenchymal spindle cell tumors (Kumar, Cotran et al. 2003).

Complete local regional control is the key treatment for surviving gastric cancer, which includes total or subtotal gastrectomy, sentinel lymph node dissection, extended lymph node dissection and resection of adjacent tissues affected by metastasis such as splenectomy and pancreatectomy (Hartgrink and van de Velde 2005; Kitagawa, Fujii et al. 2005). Out of all the patients who suffer relapse after curative surgery, up to 87.5% of them are caused by local recurrence or remained regional lymph node metastasis (Songun, Bonenkamp et al. 1996).

In a study covering 148 patients with gastro-esophageal junction (GEJ) carcinomas, it was concluded that the presence of micro metastasis in local lymph nodes is a key indicator of a poor prognosis (Heeren, Kelder et al. 2005). The current prevalent staging/diagnosis of lymph node metastasis from gastric cancer is achieved by palpation and intra-operative frozen section examination. Frozen section examination of lymph nodes can exhibit sensitivity of 74%(Kitagawa, Fujii et al. 2005).

With the appropriate gene markers, gene expression analysis and detection technology such as RT-PCR or quantitative PCR could provide an alternative method for the diagnosis or prognosis of gastric cancer. These methods have potential to provide better sensitivity and accuracy for metastasis detection test. The previously reported gene markers include CK19 (Matsuda, Kitagawa et al. 2004), Carcinoembryonic antigen (CEA), cytokeratin-20 (CK-20), and MAGE-3 (Okada, Fujiwara et al. 2001), CK18, MUC1, hTRT (Ajisaka and Miwa 2003). Unfortunately, as published data shows, most of them are not consistently expressed in all of the tumor tissues and tumor cell lines tested. Markers could not be detected by RT-PCR in 15% (CEA) to over 70% (MAGE-3) of the lymph nodes with metastasis from gastric cancer confirmed by histological examination (Okada, Fujiwara et al. 2001). CK20 was used as a marker for RT-PCR tests for disseminated cancer cells in blood from pancreas, colon, stomach, and lung carcinoma (Chausovsky, Luchansky et al. 1999; Chen, Chen et al. 2004).

Here, a combination of Markers are identified that are consistently expressed in lymph node with metastasis from gastric carcinoma at easily detectable levels which can be differentiated from their background expression in normal lymph nodes. This combination of markers is useful for the detection of disseminated carcinoma cells from gastric carcinoma, or carcinoma of similar cell origin, such as carcinoma of the esophagus, pancreas, and intestine.

### SUMMARY OF THE INVENTION

In one aspect of the invention, a set of Markers is used in combination to detect lymph node metastasis from gastric carcinoma. In lymph nodes with metastasis from gastric carcinoma, the expression levels of these Markers are distinctively higher than those of normal lymph nodes. The combination of Markers are CK19, CEA and MUC-2 markers.

In another aspect of the invention, methods of diagnosing lymph node metastasis from gastric carcinoma involve obtaining biological samples from a patient and measuring the expression levels of genes selected from a group of Markers, where the gene expression levels above pre-determined cut-off levels are indicative of metastasis from gastric carcinoma. Preferably, the biological samples are lymph nodes samples.

In another aspect of the invention, Markers are used in combination in assays to detect lymph node metastasis from gastric carcinoma.

In yet another aspect of the invention, Markers are assayed intraoperatively. The intraoperative assays can be conducted by a method that includes the steps of: preparing RNA from a lymph node that drains from the stomach; performing a quantitative RT-PCR method specific to one or more Markers and determining if the presence of the Marker exceeds a predetermined cut-off.

The present invention encompasses microarray or gene chips for performing the methods provided herein as well as kits and articles of manufacture for conducting the assays.

### DETAILED DESCRIPTION

The inventive methods, compositions, articles, and kits described and claimed in this specification include a combination of Markers. "Marker" is used throughout this specification to refer to:
a) Genes and gene expression products such as mRNA and corresponding cDNA, peptides, proteins, fragments and complements of each of the foregoing, and
b) Compositions such as probes, antibodies, ligands, haptens, and labels that, through physical or chemical interaction with a) indicate the expression of the gene or presence of the gene expression product and wherein the gene, gene expression product or compositions correspond with SEQ ID NO NM_002276, SEQ ID NO NM_002457, and SEQ ID NO M17303.

A gene corresponds to the sequence designated by a SEQ ID NO when it contains that sequence. A gene segment or fragment is also said correspond to the sequence of such gene when it contains a portion of the referenced sequence or its complement sufficient to distinguish it as being the sequence of the gene. A gene expression product is additionally said to correspond to such sequence when its RNA, mRNA, or cDNA hybridizes to the composition having such sequence (e.g. a probe) or, in the case of a peptide or protein; it is encoded by such mRNA. A segment or fragment of a gene expression product corresponds to the sequence of such gene or gene expression product when it contains a portion of the referenced gene expression product or its complement sufficient to distinguish it as being the sequence of the gene or gene expression product.

The Markers of this invention correspond to genes that encode three well-known proteins: carcinoembryonic antigen (SEQ ID NO M17303), mucin 2 (SEQ ID NO NM_002457), and cytokeratin 19 (SEQ ID NO NM_002276). Individually, the genes when used as cancer markers have not enabled an assay with acceptable performance, particularly with respect to intraoperative uses. However, in combination, as is shown in the Examples, the Markers provide sensitive and specific tests to determine gastric carcinoma metastasis. Controls are generally also assayed to ensure that a negative result does not arise from a faulty run of the assay. Constitutively expressed genes such as PBGD (SEQ ID NO NM_000190) are useful in this regard and reagents for assaying them are preferably included in kit components. PBGD is preferred because, among other things, it contains no known pseudogenes in humans, it is constitutively expressed in human tissues and it is expressed at a relatively low level and therefore is less likely to cause inhibition of the amplification of target sequences of interest.

Sample preparation generally involves working with excised tissue, preferably lymph node tissue. The distribution of metastases and micrometastases in tissues is not uniform in nodes or other tissues. Therefore, a sufficiently large sample should be obtained so that metastases will not be missed. One approach to the sampling issue in the present method is to homogenize a large tissue sample, and subsequently an appropriate portion of the homogenate is to be used to prepare RNA for molecular testing.

Preferably, the samples used in the methods of the invention are lymph node samples from lymph nodes that drain from the stomach. A useful procedure for preparing such a sample for RT-PCR assay follows; an Omni homogenizer and disposable probe for homogenization are used followed by purification of RNA with RNeasy (Qiagen) kit reagents:
a) Determine the sample weight in milligrams, if not previously recorded. Place a fresh piece of wax paper on a balance, tare, and weigh the sample. Preferably, nodes samples are greater than 30 mg.
b) Using a fresh scalpel, mince all tissue from the same node into pieces approximately 4 mm in diameter. Care is taken to avoid contamination of the tissue during processing.
c) Add homogenization (RLT) buffer to the polypropylene homogenization tube. Preferably, use 2 mL homogenization buffer per 100 mg tissue.
d) Using a clean forceps, transfer the tissue into the homogenization buffer.
e) Place a new homogenization probe onto the manual homogenizer.
f) Homogenize each node completely.
g) Process the homogenate per the RNA Purification procedures in standard Qiagen protocol, RNA is stored at -65°C or below before use.
h) Dispose of the homogenization probe.
i) Store any remaining homogenate at -65°C or below for possible later use.

Diagnostic assays can then be conducted. The Markers of this invention are useful for providing important clinical information such as whether the gastric carcinoma has metastasized to lymph node, which is indicative to the clinical staging of gastric carcinoma and the prognosis for the patient.

Collectively, this information is referred to as "Diagnostic Information" in this specification. Additionally, such Diagnostic Information includes its use as a component in providing therapy. For example, a physician having such Diagnostic Information can base his therapy (e.g., adjuvant therapy) on an indication that the cancer has metastasized to lymph nodes.

Preferred methods for assaying the Markers of the invention include determining the amount of mRNA that is produced by a gene that can code for a protein or peptide. This can be accomplished by competitive reverse transcriptase PCR (RT-PCR), real time RT-PCR, Northern Blot analysis or other tests with similar capabilities. Amplified complementary RNA (cRNA) can also be prepared from mRNA (RNA) and then applied to DNA microarrays and other evolving assay formats for testing and analysis. A number of different array configurations and methods for their production are known to those of skill in the art and are described in U.S. Patents such as: 5,445,934; 5,532,128; 5,556,752; 5,242,974; 5,384,261; 5,405,783; 5,412,087; 5,424,186; 5,429,807; 5,436,327; 5,472,672; 5,527,681; 5,529,756; 5,545,531; 5,554,501; 5,561,071; 5,571,639; 5,593,839; 5,599,695; 5,624,711; 5,658,734; and 5,700,637. The most well accepted measurements used to quantitate the results of a PCR is the Ct (threshold cycle) determination where Ct is defined as as the cycle number at which the fluorescence emission exceeds a fixed threshold. This are readily determinable according to well known procedures with algorithms for determining Ct set in the software of most PCR analyzers. An example of this is embodied in the SmartCycler PCR Instrument from Cepheid Corporation which is a preferred analytical platform.

Microarray technology allows for the measurement of the steady-state mRNA level of genes. The product of these analyses are typically measurements of the intensity of the signal received from a probe set used to detect a target sequence prepared from the sample that hybridizes to a nucleic acid sequence at a known location on the microarray. Typically, the intensity of the signal is proportional to the quantity of target prepared, and thus mRNA, expressed in the sample cells. A large number of such techniques are available and useful. Preferred methods for determining gene expression can be found in US Patents 6,271,002; 6,218,122; 6,218,114; and 6,004,755.

Analysis of the expression levels of genes can be conducted by comparing signal intensities of microarrays. This is best done by generating a ratio matrix of the expression intensities of genes in a test sample versus those in a control sample. For instance, the gene expression intensities from a diseased tissue can be compared with the expression intensities generated from benign or normal tissue of the same type. A ratio of these expression intensities indicates the fold-change in gene expression between the test and control samples. The invention also encompasses the above methods where the comparison of expression patterns is conducted with pattern recognition methods.

Levels of up and down regulation can be distinguished based on fold changes of the intensity measurements of hybridized microarray probes. A 1.5 fold difference is preferred for making such distinctions (or a p-value less than 0.05). That is, before a gene is said to be differentially expressed in diseased versus normal cells, the diseased cell is found to yield at least about 1.5 times more, or 1.5 times less intensity than the normal cells. The greater the fold difference, the more preferred is use of the gene as a diagnostic or prognostic tool. Genes selected for the combination of this invention have expression levels that result in the generation of a signal that is distinguishable from those of the normal or non-modulated genes by an amount that exceeds background using clinical laboratory instrumentation. The present invention encompasses microarrays or gene chips for performing the methods provided herein. The microarrays can contain isolated nucleic acid sequences, their complements, or portions thereof corresponding to Markers where the combination is sufficient to characterize gastric cancer or risk of relapse in a biological sample. The microarray preferably measures or characterizes at least about 1.5-fold over- or under-expression, provides a statistically significant p-value over- or under-expression, or a p-value is less than 0.05. Preferably, the microarray contains a cDNA array or an oligonucleotide array and may contain one or more internal control reagents.

The gene Markers are differentially expressed as up regulated in lymph nodes with metastasis from gastric carcinoma relative to those lymph nodes without metastasis conditions. Up regulation is a relative term meaning that a detectable difference (beyond the contribution of noise in the system used to measure it) is found in the amount of expression of the genes relative to some baseline. In this case, the baseline is the measured gene expression of makers in normal lymph nodes without metastasis from gastric carcinoma. The genes of interest in the tested lymph node samples are then either up regulated or unchanged relative to the baseline level using the same measurement method. A diagnosis or prognosis includes the determination of disease/status issues such as determining the likelihood of relapse and type of therapy. Determining the likelihood of relapse involves comparing the patterns with patterns associated with known relapse and/or known non-relapsing events or exceeding a cutoff value (usually a Ct in the case of PCR) set for such a distinction.

In some embodiments of the invention, the Marker combination of this invention can be used in conjunction with other gene-based Markers and non-genetic diagnostic methods useful in cancer diagnosis, prognosis, or treatment monitoring. For example, in some circumstances it is beneficial to combine the diagnostic power of the gene expression based methods described above with data from conventional markers such as serum protein markers (e.g., Cancer Antigen 27.29 ("CA 27.29")). This approach can be particularly useful when other testing produces ambiguous results.

The present invention encompasses methods of generating a gastric cancer diagnostic patient report and reports obtained thereby, by obtaining a biological sample from the patient; measuring gene expression of the sample; and using the results obtained thereby to generate the report. The report can also contain an assessment of patient outcome and/or probability of risk relative to the patient population.

The present invention encompasses compositions containing a microarray probe set or PCR primer/probe set corresponding to the Markers. Articles of manufacture according to the invention also include media or formatted assays used to reveal gene expression information. These can comprise, for example, microarrays in which sequence complements or probes are affixed to a matrix to which the sequences indicative of the genes of interest combine creating a readable determinant of their presence. Alternatively, articles according to the invention can be fashioned into reagent kits for conducting hybridization, amplification, and signal generation indicative of the level of expression of the genes of interest for detecting cancer.

Kits made according to the invention include formatted assays. These can include all or some of the materials needed to conduct the assays such as reagents and instructions and a medium through which nucleic acid sequences, their complements, or portions thereof are assayed. Where the assay is to be conducted in another format, the kit will have components peculiar to that format. For example, if the format is an amplification format such as PCR, Rolling Circle Amplification methods (RCA), Ligase Chain Reaction methods (LCR), Strand Displacement Amplification methods (SDA), Nucleic Acid Sequence Based Amplification methods (NASBA), then the kit will contain reagents and materials appropriate to such technologies. Optionally, the kit includes sample preparation reagents and or articles (e.g., tubes) to extract nucleic acids from tissue such as lymph node tissue. The kits may also include articles to minimize the risk of sample contamination (e.g., disposable scalpel and surface for lymph node dissection and preparation).

In a preferred kit, reagents necessary for a one-tube QRT-PCR process are included such as reverse transcriptase, a reverse transcriptase primer, a corresponding PCR primer set (for Markers and, preferably, controls), a thermostable DNA polymerase, such as Taq polymerase, and a suitable detection reagent(s), such as, without limitation, a scorpion probe, a probe for a fluorescent 5'nuclease assay, a molecular beacon probe, a single dye primer or a fluorescent dye specific to double-stranded DNA, such as ethidium bromide. The primers are preferably in quantities that yield high concentrations. Thermostable DNA polymerases are commonly and commercially available from a variety of manufacturers. Additional materials in the kit may include: suitable reaction tubes or vials, a barrier composition such as a wax bead, optionally including magnesium; reaction mixtures (typically 10X) for the reverse transcriptase and the PCR stages, including necessary buffers and reagents such as dNTPs; nuclease-or RNase-free water; RNase inhibitor; control nucleic acid (s) and/or any additional buffers, compounds, cofactors, ionic constituents, proteins and enzymes, polymers, and the like that may be used in reverse transcriptase and/or PCR stages of QRT-PCR reactions. Optionally, the kits include nucleic acid extraction reagents and materials.

The following examples are provided to illustrate but not limit the claimed invention. All references cited herein are hereby incorporated by reference herein.

### Example 1

This example is based on the use of real-time PCR. In real-time PCR, the products of the polymerase chain reaction are monitored in real-time during the exponential phase of PCR rather than by an end-point measurement. Hence, the quantification of DNA and RNA is much more precise and reproducible. Fluorescence values are recorded during every cycle and represent the amount of product amplified to that point in the amplification reaction. The more templates present at the beginning of the reaction, the fewer number of cycles it takes to reach a point in which the fluorescent signal is first recorded as statistically significant above background, which is the definition of the (Ct) values. The concept of the threshold cycle (Ct) allows for accurate and reproducible quantification using fluorescence based RT-PCR. Homogeneous detection of PCR products are preferably performed based on: (a) double- stranded DNA binding dyes (e.g., SYBR Green), (b) fluorogenic probes (e.g., Taq Man probes, Molecular Beacons), and (c) direct labeled primers (e.g., Amplifluor primers).

A total of 24 H&E metastatic positive lymph nodes and 18 H&E metastatic negative lymph node were obtained from patients diagnosed with gastric carinoma. The lymph nodes were selected as those that would drain from the stomach. 30 mg pieces of tissue were cut from each node.

A 30 mg piece of each lymph node tissue was processed by as follows:
1. The tissue piece was added to 2 ml of Buffer RLT and homogenized manually for 20-40 sec by means of a disposable tissue grinder.
2. Mix 400 µL of homogenate with 400 µL of 70% ethanol in a 4.5 mL tube by vortexing for 10 seconds.
3. For each sample, attach a VacValve onto a Vacuum Manifold, and a disposable VacConnector to each valve.
4. Attach a RNeasy mini column on to the VacConnector, leaving the cap open.
5. Aliquot 700 µL homogenate/ethanol mix from step 2 onto the RNA Spin Column.
6. Turn VacValves to the On position and apply vacuum (800-1200 mbars) until sample is filtered (approximately 30 seconds).
7. Stop vacuum; add 700 µL of Wash Buffer 1 to the Column. Start vacuum and allow the solution to filter through the Column. Stop vacuum.
8. Add 700 µL of Wash Buffer 2 to the Column. Start vacuum and allow the solution to filter through the column. Stop vacuum.
9. Remove each Column from the Vacuum Manifold and place into a 2 mL collection tube (supplied with column).
10. Centrifuge the tubes containing the RNA Spin Columns for 30 seconds at >10,000 RPM in a microcentrifuge.
11. Discard the collection tube. Put the Column into a new fresh collection tube (1.5-1.7 mL polypropylene microcentrifuge tube).
12. Add 50 µL of RNase-free water directly to the filter membrane of Column.
13. Centrifuge at >10,000 RPM for 30 seconds in a microcentrifuge.
14. Before continuing to step 14, ensure that the collection tube has been labeled in such a way that the source specimen can be identified (example: patient ID& Node ID).
15. Discard the Column. Approximately 50 µL of eluted RNA solution will be contained in the collection tube.

Extracted RNA was subjected to one-step RT-PCR amplification in a Cepheid Smart Cycler II. The reaction contains the following components: 50 mM Bicine / KOH, pH 8.2, 115 mM Potassium Acetate,
8% v/v Glycerol, 0.2 mg/ml BSA, 150 mM Trehalose, 0.2% v/v Tween 20, 0.2 mM Tris-Cl pH 8, 3.5 mM MnSO4 or Mn
(acetate), 0.3 mM dATP, 0.3 mM dCTP, 0.3 mM dGTP, 0.3 mM TTP, 100 U/ml Tth, 20 ug/ml Antibody TP6-25.3, 0.4-0.8
uM each probe and primer. Probe and primer sequences are designed such that nonspecific priming events are
minimized and such that they are compatible with multiplexed amplification / detection with the following rapid RT-PCR profile:
95 C for 3 sec, 60 C for 3 min, then 32 cycles of: 95 C for 1 sec, 65 C for 6 sec* and 72 C for 3 sec - total amplification time = 16 min.

The primers and probes used in this example were as follows:
MUC2:
   Upper primer: ATGAACTACGCTCCTGGCTT (SEQ ID NO: 5 : NM_002457)
   Lower primer: AAACTCTCTGGGCACATTGTC (SEQ ID NO: 6 : NM_002457)
   Probe: FAM-CGTGAAGACCTGCGGCTGTGT-BHQ1-TT (SEQ ID NO: 7 : NM_002457)
CEA:
   Upper primer: ACCACAGTCACGACGATCACA (SEQ ID NO: 8 : M17303)
   Lower primer: CGGGGTTGGAGTTGTTGCT (SEQ ID NO: 9: M17303)
   Probe: TexasRed-CTATGCAGAGCCACCCAAACCCTT-BHQ2-TT (SEQ ID NO: 10 : M17303)
CK19:
   Upper primer: CACCCTTCAGGGTCTTGAGATT (SEQ ID NO: 11)
   Lower primer: TCCGTTTCTGCCAGTGTGTC (SEQ ID NO: 12 )
   Probe: 5' Quasar 570 d(ACAGCTGAGCATGAAAGCTGCCTT)-BHQ-2-TT) 3' (SEQ ID NO: 13)
PBGD:
   Primer 1 CTGCTTCGCTGCATCGCTGAAA (SEQ ID NO: 14 )
   Primer 2 CAGACTCCTCCAGTCAGGTACA (SEQ ID NO: 15)
   Probe - FAM-CCTGAGGCACCTGGAAGGAGGCTGCAGTGT-TAMRA (SEQ ID NO: 16)

The samples and the assay results were summarized in Table 1. The resulting Ct values were utilized to determine relative expression of the genes across all samples tested. The Ct value established was 27 for the Markers and 35 for the internal control (PBGD). Samples with a Ct of 27 or lower for any of the 3 markers are considered positive for metastasis, while samples with Ct values above 27 for all 3 markers are considered negative for metastasis. The Ct value for internal control needs to be lower than 35 for the negative diagnosis result to be valid.Based on these cut-off values, the assay was used to correctly evaluate 17 out of the 18 negative lymph nodes (94.4% specificity) and all 24 of the positive lymph nodes (100% sensitivity).

**Table 1. Detection of lymph node metastasis from patients with gastric adenocarcinoma**

| Sample | Lymph Node Diagnosis | IC Ct | MUC Ct | CK19 Ct | CEA Ct |
|---|---|---|---|---|---|
| 1 | Normal | 25.65 | 36.6 | 27.25 | 27.9 |
| 2 | Normal | 25.45 | 30.6 | 18.8 | 23.15 |
| 3 | Normal | 23.7 | 32.8 | 31.2 | 33.5 |
| 4 | Normal | 24.8 | 40 | 40 | 40 |
| 5 | Normal | 25.2 | 40 | 33.3 | 40 |
| 6 | Normal | 24.4 | 40 | 31.7 | 40 |
| 7 | Normal | 24.8 | 31.8 | 30.5 | 33.9 |
| 8 | Normal | 25.2 | 40 | 31.2 | 40 |
| 9 | Normal | 26.1 | 35.4 | 40 | 40 |
| 10 | Normal | 27.4 | 34.5 | 34.6 | 40 |
| 11 | Normal | 24.7 | 28.2 | 28 | 28.9 |
| 12 | Normal | 25.7 | 27.3 | 28.5 | 29.1 |
| 13 | Normal | 25.8 | 40 | 32.8 | 32.7 |
| 14 | Normal | 26.2 | 40 | 31 | 40 |
| 15 | Normal | 19.2 | 40 | 37.4 | 40 |
| 16 | Normal | 26.4 | 40 | 35.7 | 40 |
| 17 | Normal | 27.2 | 40 | 32.9 | 40 |
| 18 | Normal | 26.3 | 40 | 32.7 | 40 |
| 19 | Metastatic | 23.6 | 23.9 | 21.05 | 21.35 |
| 20 | Metastatic | 23.85 | 19.05 | 19.65 | 19.5 |
| 21 | Metastatic | 21.15 | 21 | 19.3 | 19.2 |
| 22 | Metastatic | 26.1 | 22.45 | 20.1 | 20.7 |
| 23 | Metastatic | 25.25 | 24.8 | 18.65 | 20.35 |
| 24 | Metastatic | 31.85 | 26.7 | 24.75 | 24.4 |
| 25 | Metastatic | 30.3 | 26.25 | 23.45 | 25.65 |
| 26 | Metastatic | 26.7 | 22.1 | 20.95 | 23.9 |
| 27 | Metastatic | 27.7 | 24.55 | 23.5 | 28.3 |
| 28 | Metastatic | 26.15 | 32.6 | 25.55 | 30.3 |
| 29 | Metastatic | 25.85 | 24.95 | 19.55 | 24.45 |
| 30 | Metastatic | 25.75 | 24.65 | 19.45 | 23.8 |
| 31 | Metastatic | 29.05 | 22.85 | 18.5 | 21.5 |
| 32 | Metastatic | 27.7 | 24.95 | 20.2 | 24.25 |
| 33 | Metastatic | 27.8 | 27 | 21.5 | 21.05 |
| 34 | Metastatic | 25.15 | 25.25 | 25.8 | 19.95 |
| 35 | Metastatic | 26.6 | 23.05 | 23.5 | 22.1 |
| 36 | Metastatic | 27.65 | 32.55 | 17.05 | 16.05 |
| 37 | Metastatic | 24.95 | 26.35 | 19.25 | 17.35 |
| 38 | Metastatic | 24.75 | 25.4 | 18.2 | 15.2 |
| 39 | Metastatic | 25.8 | 28 | 21.85 | 19.3 |
| 40 | Metastatic | 28.1 | 31.95 | 20.35 | 18.15 |
| 41 | Metastatic | 25.25 | 18.8 | 19.45 | 18.05 |
| 42 | Metastatic | 25 | 32.9 | 19.2 | 19.5 |

## Claims

1. A method of detecting lymph node metastasis from gastric carcinoma comprising the steps:
a. obtaining a biological sample from a patient, and
b. measuring Marker levels in the sample;
wherein the levels above the pre-determined cut-off levels are indicative of lymph node metastasis from gastric carcinoma.

2. The method of claim 1 wherein the Markers are genes, gene fragments, or complements thereof.

3. The method of claim 1 wherein the Markers are proteins, protein fragments, or polypeptides.

4. The method of claim 1 further comprising the use of another diagnostic method.

5. The method of claim 4 wherein the other diagnostic method comprises conducting a serum assay.

6. The method of claim 1 further comprising the measurement of a consitutively expressed gene.

7. The method of claim 1 having a sensitivity of at least 80%.

8. The method of claim 1 having a specificity of at least 70%.

9. The method of claim 1 wherein the sample comprises lymph node tissue.

10. A method of determining or adjusting therapy for a gastric carcinoma patient comprising the steps:
a. obtaining a biological sample from a gastric cancer patient; and
b. measuring the levels in the sample of a Marker;
wherein levels above or below a cut-off levels are consistent with a course of disease or physical condition for which a therapy is determined or adjusted.

11. The method of claim 10 wherein the Marker is a gene, a gene fragment, or the complement thereof.

12. The method of claim 10 wherein the Marker is a protein, protein fragment, or polypeptide.

13. The method of claim 10 further comprising the measurement of a consitutively expressed gene.

14. The method of claim 10 wherein the specificity is at least 70%.

15. The method of claim 10 wherein the sensitivity is at least about 80%.

16. The method of claim 1 or claim 10 conducted with a microarray.

17. The method of claim 1 conducted by using an amplification process.

18. The method of claim 17 wherein the amplification process is a PCR.

19. The method of claim 18 wherein the PCR s an RT-PCR.

20. The method of claim 10 conducted by using an amplification process.

21. A kit for conducting an assay to determine gastric cancer diagnosis in a biological sample comprising materials for detecting a Marker.

22. The kit of claim 21 including a microarray and reagents.

23. The kit of claim 21 including PCR reagents.

24. The kit of claim 21 including instructions.

25. The kit of claim 21 wherein the Markers consist of CEA, MUC2, and CK-19.

26. A method of detecting lymph node metastasis from gastric carcinoma comprising the steps:
a. obtaining a biological sample from a patient, and
b. measuring Marker levels in the sample;
wherein the levels above the pre-determined cut-off levels are indicative of lymph node metastasis from gastric carcinoma and wherein the Markers consist of CEA, MUC2, and CK-19.

27. The method of claim 26 further comprising the use of another diagnostic method.

28. The method of claim 26 wherein the other diagnostic method comprises conducting a serum assay.

29. The method of claim 26 further comprising the measurement of a consitutively expressed gene.

30. The method of claim 26 having a sensitivity of at least 80%.

31. The method of claim 26 having a specificity of at least 70%.


### Annex to the application documents - subsequently filed sequences listing

SEQUENCE LISTING

<110>  VERIDEX, LLC

<120>  DETECTION OF LYMPH NODE METASTASIS FROM GASTRIC CARCINOMA

<130>  P047224EP

<140>  EP 07252244.4
<141>  2007-06-04

<150>  US 11/447255
<151>  2006-06-05

<160>  16

<170>  PatentIn version 3.3

<210>  1
<211>  3036
<212>  DNA
<213>  Homo sapiens


<220>
<221>  misc_feature
<222>  (1)..(79)
<223>  >gi|178676|gb|M17303.1|HUMANTCE Human carcinoembryonic antigen
       gene, complete cds

<400>  1
cgaccagcag accagacagt cacagcagcc ttgacaaaac gttcctggaa ctcaagcact      60

tctccacaga ggaggacaga gcagacagca gagaccatgg agtctccctc ggcccctccc     120

cacagatggt gcatcccctg gcagaggctc ctgctcacag cctcacttct aaccttctgg     180

aacccgccca ccactgccaa gctcactatt gaatccacgc cgttcaatgt cgcagagggg     240

aaggaggtgc ttctacttgt ccacaatctg ccccagcatc tttttggcta cagctggtac     300

aaaggtgaaa gagtggatgg caaccgtcaa attataggat atgtaatagg aactcaacaa     360

gctaccccag ggcccgcata cagtggtcga gagataatat accccaatgc atccctgctg     420

atccagaaca tcatccagaa tgacacagga ttctacaccc tacacgtcat aaagtcagat     480

cttgtgaatg aagaagcaac tggccagttc cgggtatacc cggagctgcc caagccctcc     540

atctccagca acaactccaa acccgtggag gacaaggatg ctgtggcctt cacctgtgaa     600

cctgagactc aggacgcaac ctacctgtgg tgggtaaaca atcagagcct cccggtcagt     660

cccaggctgc agctgtccaa tggcaacagg accctcactc tattcaatgt cacaagaaat     720

gacacagcaa gctacaaatg tgaaacccag aacccagtga gtgccaggcg cagtgattca     780

gtcatcctga atgtcctcta tggcccggat gcccccacca tttcccctct aaacacatct     840

tacagatcag gggaaaatct gaacctctcc tgccatgcag cctctaaccc acctgcacag     900

tactcttggt ttgtcaatgg gactttccag caatccaccc aagagctctt tatccccaac     960

atcactgtga ataatagtgg atcctatacg tgccaagccc ataactcaga cactggcctc    1020

aataggacca cagtcacgac gatcacagtc tatgcagagc cacccaaacc cttcatcacc    1080

agcaacaact ccaaccccgt ggaggatgag gatgctgtag ccttaacctg tgaacctgag    1140

attcagaaca caacctacct gtggtgggta aataatcaga gcctcccggt cagtcccagg 1200
ctgcagctgt ccaatgacaa caggaccctc actctactca gtgtcacaag gaatgatgta 1260
ggaccctatg agtgtggaat ccagaacgaa ttaagtgttg accacagcga cccagtcatc 1320
ctgaatgtcc tctatggccc agacgacccc accatttccc cctcatacac ctattaccgt 1380
ccaggggtga acctcagcct ctcctgccat gcagcctcta acccacctgc acagtattct 1440
tggctgattg atgggaacat ccagcaacac acacaagagc tctttatctc caacatcact 1500
gagaagaaca gcggactcta tacctgccag gccaataact cagccagtgg ccacagcagg 1560
actacagtca agacaatcac agtctctgcg gagctgccca agccctccat ctccagcaac 1620
aactccaaac ccgtggagga caaggatgct gtggccttca cctgtgaacc tgaggctcag 1680
aacacaacct acctgtggtg ggtaaatggt cagagcctcc cagtcagtcc caggctgcag 1740
ctgtccaatg gcaacaggac cctcactcta ttcaatgtca caagaaatga cgcaagagcc 1800
tatgtatgtg gaatccagaa ctcagtgagt gcaaaccgca gtgacccagt caccctggat 1860
gtcctctatg ggccggacac ccccatcatt tcccccccag actcgtctta cctttcggga 1920
gcgaacctca acctctcctg ccactcggcc tctaacccat ccccgcagta ttcttggcgt 1980
atcaatggga taccgcagca acacacacaa gttctcttta tcgccaaaat cacgccaaat 2040
aataacggga cctatgcctg ttttgtctct aacttggcta ctggccgcaa taattccata 2100
gtcaagagca tcacagtctc tgcatctgga acttctcctg gtctctcagc tggggccact 2160
gtcggcatca tgattggagt gctggttggg gttgctctga tatagcagcc ctggtgtagt 2220
ttcttcattt caggaagact gacagttgtt ttgcttcttc cttaaagcat ttgcaacagc 2280
tacagtctaa aattgcttct ttaccaagga tatttacaga aaagactctg accagagatc 2340
gagaccatcc tagccaacat cgtgaaaccc catctctact aaaaatacaa aaatgagctg 2400
ggcttggtgg cgcgcacctg tagtcccagt tactcgggag gctgaggcag gagaatcgct 2460
tgaacccggg aggtggagat tgcagtgagc ccagatcgca ccactgcact ccagtctggc 2520
aacagagcaa gactccatct caaaaagaaa agaaaagaag actctgacct gtactcttga 2580
atacaagttt ctgataccac tgcactgtct gagaatttcc aaaactttaa tgaactaact 2640
gacagcttca tgaaactgtc caccaagatc aagcagagaa aataattaat ttcatgggac 2700
taaatgaact aatgaggata atattttcat aattttttat ttgaaatttt gctgattctt 2760
taaatgtctt gtttcccaga tttcaggaaa ctttttttct tttaagctat ccacagctta 2820
cagcaatttg ataaaatata cttttgtgaa caaaaattga gacatttaca ttttctccct 2880
atgtggtcgc tccagacttg ggaaactatt catgaatatt tatattgtat ggtaatatag 2940
ttattgcaca agttcaataa aaatctgctc tttgtatgac agaatacatt tgaaaacatt 3000
ggttatatta ccaagacttt gactagaatg tcgtat 3036

<210> 2
<211> 1513
<212> DNA
<213> Homo sapiens

<220>
<221>  misc_feature
<222>  (1)..(65)
<223>  >gi|40217850|ref|NM_002276.3| Homo sapiens keratin 19 (KRT19),
      mRNA

<400>  2
cgcccctgac accattcctc ccttcccccc tccaccggcc gcgggcataa aaggcgccag      60

gtgagggcct cgccgctcct cccgcgaatc gcagcttctg agaccagggt tgctccgtcc     120

gtgctccgcc tcgccatgac ttcctacagc tatcgccagt cgtcggccac gtcgtccttc     180

ggaggcctgg gcggcggctc cgtgcgtttt gggccggggg tcgcctttcg cgcgcccagc     240

attcacgggg gctccggcgg ccgcggcgta tccgtgtcct ccgcccgctt tgtgtcctcg     300

tcctcctcgg gggcctacgg cggcggctac ggcggcgtcc tgaccgcgtc cgacgggctg     360

ctggcgggca acgagaagct aaccatgcag aacctcaacg accgcctggc ctcctacctg     420

gacaaggtgc gcgccctgga ggcggccaac ggcgagctag aggtgaagat ccgcgactgg     480

taccagaagc aggggcctgg gccctcccgc gactacagcc actactacac gaccatccag     540

gacctgcggg acaagattct tggtgccacc attgagaact ccaggattgt cctgcagatc     600

gacaatgccc gtctggctgc agatgacttc cgaaccaagt ttgagacgga acaggctctg     660

cgcatgagcg tggaggccga catcaacggc ctgcgcaggg tgctggatga gctgaccctg     720

gccaggaccg acctggagat gcagatcgaa ggcctgaagg aagagctggc ctacctgaag     780

aagaaccatg aggaggaaat cagtacgctg aggggccaag tgggaggcca ggtcagtgtg     840

gaggtggatt ccgctccggg caccgatctc gccaagatcc tgagtgacat gcgaagccaa     900

tatgaggtca tggccgagca gaaccggaag gatgctgaag cctggttcac cagccggact     960

gaagaattga accgggaggt cgctggccac acggagcagc tccagatgag caggtccgag    1020

gttactgacc tgcggcgcac ccttcagggt cttgagattg agctgcagtc acagctgagc    1080

atgaaagctg ccttggaaga cacactggca gaaacggagg cgcgctttgg agcccagctg    1140

gcgcatatcc aggcgctgat cagcggtatt gaagcccagc tgggcgatgt gcgagctgat    1200

agtgagcggc agaatcagga gtaccagcgg ctcatggaca tcaagtcgcg gctggagcag    1260

gagattgcca cctaccgcag cctgctcgag ggacaggaag atcactacaa caatttgtct    1320

gcctccaagg tcctctgagg cagcaggctc tggggcttct gctgtccttt ggagggtgtc    1380

ttctgggtag agggatggga aggaagggac ccttaccccc ggctcttctc ctgacctgcc    1440

aataaaaatt tatggtccaa gggaaaaaaa aaaaaaaaaa aaaaaaaaaa aaaaaaaaaa    1500

aaaaaaaaaa aaa                                                       1513


<210>  3
<211>  15720
<212>  DNA
<213>  Homo sapiens


<220>

<221>  misc_feature
<222>  (1)..(83)
<223>  >gi|4505284|ref|NM_002457.1| Homo sapiens mucin 2,
       intestinal/tracheal (MUC2), mRNA

<400>  3
caacccacac cgcccctgcc agccaccatg gggctgccac tagcccgcct ggcggctgtg      60

tgcctggccc tgtctttggc agggggctcg gagctccaga cagagggcag aacccgatac     120

cacggccgca acgtctgcag cacctggggc aacttccact acaagacctt cgacggggac     180

gtcttccgct tccccggcct ctgcgactac aacttcgcct ccgactgccg aggctcctac     240

aaggaatttg ctgtgcacct gaagcggggt ccgggccagg ctgaggcccc cgccggggtg     300

gagtccatcc tgctgaccat caaggatgac accatctacc tcacccgcca cctggctgtg     360

cttaacgggg ccgtggtcag caccccgcac tacagccccg ggctgctcat tgagaagagc     420

gatgcctaca ccaaagtcta ctcccgcgcc ggcctcaccc tcatgtggaa ccgggaggat     480

gcactcatgc tggagctgga cactaagttc cggaaccaca cctgtggcct ctgcggggac     540

tacaacggcc tgcagagcta ttcagaattc ctctctgacg gcgtgctctt cagtcccctg     600

gagtttggga acatgcagaa gatcaaccag cccgatgtgg tgtgtgagga tcccgaggag     660

gaggtggccc ccgcatcctg ctccgagcac cgcgccgagt gtgagaggct gctgaccgcc     720

gaggccttcg cggactgtca ggacctggtg ccgctggagc cgtatctgcg cgcctgccag     780

caggaccgct gccggtgccc gggcggtgac acctgcgtct gcagcaccgt ggccgagttc     840

tcccgccagt gctcccacgc cggcggccgg cccgggaact ggaggaccgc cacgctctgc     900

cccaagacct gccccgggaa cctggtgtac ctggagagcg gctcgccctg catggacacc     960

tgctcacacc tggaggtgag cagcctgtgc gaggagcacc gcatggacgg ctgtttctgc    1020

ccagaaggca ccgtatatga cgacatcggg gacagtggct gcgttcctgt gagccagtgc    1080

cactgcaggc tgcacggaca cctgtacaca ccgggccagg agatcaccaa tgactgcgag    1140

cagtgtgtct gtaacgctgg ccgctgggtg tgcaaagacc tgccctgccc cggcacctgt    1200

gccctggaag gcggctccca catcaccacc ttcgatggga agacgtacac cttccacggg    1260

gactgctact atgtcctggc caagggtgac cacaacgatt cctacgctct cctgggcgag    1320

ctggccccct gtggctccac agacaagcag acctgcctga agacggtggt gctgctggct    1380

gacaagaaga agaatgcggt ggtcttcaag tccgatggca gtgtactgct caaccagctg    1440

caggtgaacc tgccccacgt gaccgcgagc ttctctgtct tccgcccgtc ttcctaccac    1500

atcatggtga gcatggccat tggcgtccgg ctgcaggtgc agctggcccc agtcatgcaa    1560

ctctttgtga cactggacca ggcctcccag gggcaggtgc agggcctctg cgggaacttc    1620

aacggcctgg aaggtgacga cttcaagacg gccagcgggc tggtggaggc cacgggggcc    1680

ggctttgcca acacctggaa ggcacagtca acctgccatg acaagctgga ctggttggac    1740

gatccctgct ccctgaacat cgagagcgcc aactacgccg agcactggtg ctccctcctg    1800

aagaagacag agaccccctt tggcaggtgc cactcggctg tggaccctgc tgagtattac    1860

aagaggtgca aatatgacac gtgtaactgt cagaacaatg aggactgcct gtgcgccgcc    1920

ctgtcctcct acgcgcgcgc ctgcaccgcc aagggcgtca tgctgtgggg ctggcgggag 1980
catgtctgca acaaggatgt gggctcctgc cccaactcgc aggtcttcct gtacaacctg 2040
accacctgcc agcagacctg ccgctccctc tccgaggccg acagccactg tctcgagggc 2100
tttgcgcctg tggacggctg cggctgccct gaccacacct tcctggacga gaagggccgc 2160
tgcgtacccc tggccaagtg ctcctgttac caccgcggtc tctacctgga ggcgggggat 2220
gtggtcgtca ggcaggaaga acgatgtgtg tgccgggatg ggcggctgca ctgtaggcag 2280
atccggctga tcggccagag ctgcacggcc ccaaagatcc acatggactg cagcaacctg 2340
actgcactgg ccacctcgaa gccccgagcc ctcagctgcc agacgctggc cgccggctat 2400
taccacacag agtgtgtcag tggctgtgtg tgccccgacg ggctgatgga tgacggccgg 2460
ggtggctgcg tggtggagaa ggaatgccct tgcgtccata acaacgacct gtattcttcc 2520
ggcgccaaga tcaaggtgga ctgcaatacc tgcacctgca agagaggacg ctgggtgtgc 2580
acccaggctg tgtgccatgg cacctgctcc atttacggga gtggccacta catcaccttt 2640
gatgggaagt actacgactt tgacggacac tgctcctacg tggctgttca ggactactgc 2700
ggccagaact cctcactggg ctcattcagc atcatcaccg agaacgtccc ctgtggcact 2760
acgggcgtca cctgctccaa ggccatcaag atcttcatgg ggaggacgga gctgaagttg 2820
gaagacaagc accgtgtggt gatccagcgt gatgagggtc accacgtggc ctacaccacg 2880
cgggaggtgg gccagtacct ggtggtggag tccagcacgg gcatcatcgt catctgggac 2940
aagaggacca ccgtgttcat caagctggct ccctcctaca agggcaccgt gtgtggcctg 3000
tgtgggaact ttgaccaccg ctccaacaac gacttcacca cgcgggacca catggtggtg 3060
agcagcgagc tggacttcgg gaacagctgg aaggaggccc ccacctgccc agatgtgagc 3120
accaaccccg agccctgcag cctgaacccg caccgccgct cctgggccga gaagcagtgc 3180
agcatcctca aaagcagcgt gttcagcatc tgccacagca aggtggaccc caagcccttc 3240
tacgaggcct gtgtgcacga ctcgtgctcc tgtgacacgg gtggggactg tgagtgcttc 3300
tgctctgccg tggcctccta cgcccaggag tgtaccaaag agggggcctg cgtgttctgg 3360
aggacgccgg acctgtgccc catattctgc gactactaca accctccgca tgagtgtgag 3420
tggcactatg agccatgtgg gaaccggagc ttcgagacct gcaggaccat caacggcatc 3480
cactccaaca tctccgtgtc ctacctggag ggctgctacc cccggtgccc caaggacagg 3540
cccatctatg aggaggatct gaagaagtgt gtcactgcag acaagtgtgg ctgctatgtc 3600
gaggacaccc actacccacc tggagcatcg gttcccaccg aggagacctg caagtcctgc 3660
gtgtgtacca actcctccca agtcgtctgc aggccggagg aaggaaagat tcttaaccag 3720
acccaggatg gcgccttctg ctactgggag atctgtggcc ccaacgggac ggtggagaag 3780
cacttcaaca tctgttccat tacgacacgc ccgtccaccc tgaccacctt caccaccatc 3840
accctcccca ccacccccac ctccttcacc actaccacca ccaccaccac cccgacctcc 3900
agcacagttt tatcaacaac tccgaagctg tgctgcctct ggtctgactg gatcaatgag 3960

gaccacccca gcagtggcag cgacgacggt gaccgagaac catttgatgg ggtctgcggg 4020
gcccctgagg acatcgagtg caggtcggtc aaggatcccc acctcagctt ggagcagcat 4080
ggccagaagg tgcagtgtga tgtctctgtt gggttcattt gcaagaatga agaccagttt 4140
ggaaatggac catttggact gtgttacgac tacaagatac gtgtcaattg ttgctggccc 4200
atggataagt gtatcaccac tcccagccct ccaactacca ctcccagccc tccaccaacc 4260
acgacgacca cccttccacc aaccaccacc cccagccctc caaccaccac cacaaccacc 4320
cctccaccaa ccaccacccc cagccctcca ataaccacca cgaccacccc tctaccaacc 4380
accactccca gccctccaat aagcaccaca accacccctc caccaaccac cactcccagc 4440
cctccaacca ccactcccag ccctccaacc accactccca gccctccaac aaccaccaca 4500
accacccctc caccaaccac cactcccagc cctccaatga ctacgcccat cactccacca 4560
gccagcacta ccacccttcc accaaccacc actcccagcc ctccaacaac caccacaacc 4620
acccctccac caaccaccac tcccagtcct ccaacgacta cgcccatcac tccaccaacc 4680
agcactacta cccttccacc aaccaccact cccagccctc caccaaccac cacaaccacc 4740
cctccaccaa ccaccactcc cagccctcca acaaccacca ctcccagtcc tccaacaatc 4800
accacaacca cccctccacc aaccaccact cccagccctc caacaacgac cacaaccacc 4860
cctccaccaa ccaccactcc cagccctcca acgactacac ccatcactcc accaaccagc 4920
actaccaccc ttccaccaac caccactccc agccctccac caaccaccac aaccacccct 4980
ccaccaacca ccactcccag ccctccaaca accaccactc ccagccctcc aataaccacc 5040
acaaccaccc ctccaccaac caccactccc agctctccaa taaccaccac tcccagccct 5100
ccaacaacca ccatgaccac cccttcacca accaccaccc ccagctctcc aataaccacc 5160
acaaccaccc cttcctcaac taccactccc agccctccac caaccaccat gaccacccct 5220
tcaccaacca ccactcccag ccctccaaca accaccatga ccacccttcc accaaccacc 5280
acttccagcc ctctaacaac tactcctcta cctccatcaa taactcctcc tacattttca 5340
ccattctcaa cgacaacccc tactacccca tgcgtgcctc tctgcaattg gactggctgg 5400
ctggattctg gaaaacccaa ctttcacaaa ccaggtggag acacagaatt gattggagac 5460
gtctgtggac caggctgggc agctaacatc tcttgcagag ccaccatgta tcctgatgtt 5520
cccattggac agcttggaca aacagtggtg tgtgatgtct ctgtggggct gatatgcaaa 5580
aatgaagacc aaaagccagg tggggtcatc cctatggcct tctgcctcaa ctacgagatc 5640
aacgttcagt gctgtgagtg tgtcacccaa cccaccacca tgacaaccac caccacagag 5700
aacccaactc cgccaaccac gacacccatc accaccacca ctacggtgac cccaacccca 5760
acacccaccg gcacacagac cccaaccacg acacccatca ccaccaccac tacggtgacc 5820
ccaaccccaa cacccaccgg cacacagacc ccaaccacga cacccatcac caccaccact 5880
acggtgaccc caaccccaac acccaccggc acacagaccc caaccacgac acccatcacc 5940
accaccacta cggtgacccc aaccccaaca cccaccggca cacagacccc aaccacgaca 6000
cccatcacca ccaccactac ggtgacccca accccaacac ccaccggcac acagacccca 6060

accacgacac ccatcaccac caccactacg gtgaccccaa ccccaacacc caccggcaca 6120
cagaccccaa ccacgacacc catcaccacc accactacgg tgaccccaac cccaacaccc 6180
accggcacac agaccccaac cacgacaccc atcaccacca ccactacggt gaccccaacc 6240
ccaacaccca ccggcacaca gaccccaacc acgacaccca tcaccaccac cactacggtg 6300
accccaaccc caacacccac cggcacacag accccaacca cgacacccat caccaccacc 6360
actacggtga ccccaacccc aacacccacc ggcacacaga ccccaaccac gacacccatc 6420
accaccacca ctacggtgac cccaacccca acacccaccg gcacacagac cccaaccacg 6480
acacccatca ccaccaccac tacggtgacc ccaaccccaa cacccaccgg cacacagacc 6540
ccaaccacga cacccatcac caccaccact acggtgaccc caaccccaac acccaccggc 6600
acacagaccc caaccacgac acccatcacc accaccacta cggtgacccc aaccccaaca 6660
cccaccggca cacagacccc aaccacgaca cccatcacca ccaccactac ggtgacccca 6720
accccaacac ccaccggcac acagacccca accacgacac ccatcaccac caccactacg 6780
gtgaccccaa ccccaacacc caccggcaca cagaccccaa ccacgacacc catcaccacc 6840
accactacgg tgaccccaac cccaacaccc accggcacac agaccccaac cacgacaccc 6900
atcaccacca ccactacggt gaccccaacc ccaacaccca ccggcacaca gaccccaacc 6960
acgacaccca tcaccaccac cactacggtg accccaaccc caacacccac cggcacacag 7020
accccaacca cgacacccat caccaccacc actacggtga ccccaacccc aacacccacc 7080
ggcacacaga ccccaaccac gacacccatc accaccacca ctacggtgac cccaacccca 7140
acacccaccg gcacacagac cccaaccacg acacccatca ccaccaccac tacggtgacc 7200
ccaaccccaa cacccaccgg cacacagacc ccaaccacga cacccatcac caccaccact 7260
acggtgaccc caaccccaac acccaccggc acacagaccc caaccacgac acccatcacc 7320
accaccacta cggtgacccc aaccccaaca cccaccggca cacagacccc aaccacgaca 7380
cccatcacca ccaccactac ggtgacccca accccaacac ccaccggcac acagacccca 7440
accacgacac ccatcaccac caccactacg gtgaccccaa ccccaacacc caccggcaca 7500
cagaccccaa ccacgacacc catcaccacc accactacgg tgaccccaac cccaacaccc 7560
accggcacac agaccccaac cacgacaccc atcaccacca ccactacggt gaccccaacc 7620
ccaacaccca ccggcacaca gaccccaacc acgacaccca tcaccaccac cactacggtg 7680
accccaaccc caacacccac cggcacacag accccaacca cgacacccat caccaccacc 7740
actacggtga ccccaacccc aacacccacc ggcacacaga ccccaaccac gacacccatc 7800
accaccacca ctacggtgac cccaacccca acacccaccg gcacacagac cccaaccacg 7860
acacccatca ccaccaccac tacggtgacc ccaaccccaa cacccaccgg cacacagacc 7920
ccaaccacga cacccatcac caccaccact acggtgaccc caaccccaac acccaccggc 7980
acacagaccc caaccacgac acccatcacc accaccacta cggtgacccc aaccccaaca 8040
cccaccggca cacagacccc aaccacgaca cccatcacca ccaccactac ggtgacccca 8100

accccaacac ccaccggcac acagacccca accacgacac ccatcaccac caccactacg 8160
gtgaccccaa ccccaacacc caccggcaca cagaccccaa ccacgacacc catcaccacc 8220
accactacgg tgaccccaac cccaacaccc accggcacac agaccccaac cacgacaccc 8280
atcaccacca ccactacggt gaccccaacc ccaacaccca ccggcacaca gaccccaacc 8340
acgacaccca tcaccaccac cactacggtg accccaaccc caacacccac cggcacacag 8400
accccaacca cgacacccat caccaccacc actacggtga ccccaacccc aacacccacc 8460
ggcacacaga ccccaaccac gacacccatc accaccacca ctacggtgac cccaacccca 8520
acacccaccg gcacacagac cccaaccacg acacccatca ccaccaccac tacggtgacc 8580
ccaaccccaa cacccaccgg cacacagacc ccaaccacga cacccatcac caccaccact 8640
acggtgaccc caaccccaac acccaccggc acacagaccc caaccacgac acccatcacc 8700
accaccacta cggtgacccc aaccccaaca cccaccggca cacagacccc aaccacgaca 8760
cccatcacca ccaccactac ggtgacccca accccaacac ccaccggcac acagacccca 8820
accacgacac ccatcaccac caccactacg gtgaccccaa ccccaacacc caccggcaca 8880
cagaccccaa ccacgacacc catcaccacc accactacgg tgaccccaac cccaacaccc 8940
accggcacac agaccccaac cacgacaccc atcaccacca ccactacggt gaccccaacc 9000
ccaacaccca ccggcacaca gaccccaacc acgacaccca tcaccaccac cactacggtg 9060
accccaaccc caacacccac cggcacacag accccaacca cgacacccat caccaccacc 9120
actacggtga ccccaacccc aacacccacc ggcacacaga ccccaaccac gacacccatc 9180
accaccacca ctacggtgac cccaacccca acacccaccg gcacacagac cccaaccacg 9240
acacccatca ccaccaccac tacggtgacc ccaaccccaa cacccaccgg cacacagacc 9300
ccaaccacga cacccatcac caccaccact acggtgaccc caaccccaac acccaccggc 9360
acacagaccc caaccacgac acccatcacc accaccacta cggtgacccc aaccccaaca 9420
cccaccggca cacagacccc aaccacgaca cccatcacca ccaccactac ggtgacccca 9480
accccaacac ccaccggcac acagacccca accacgacac ccatcaccac caccactacg 9540
gtgaccccaa ccccaacacc caccggcaca cagaccccaa ccacgacacc catcaccacc 9600
accactacgg tgaccccaac cccaacaccc accggcacac agaccccaac cacgacaccc 9660
atcaccacca ccactacggt gaccccaacc ccaacaccca ccggcacaca gaccccaacc 9720
acgacaccca tcaccaccac cactacggtg accccaaccc caacacccac cggcacacag 9780
accccaacca cgacacccat caccaccacc actacggtga ccccaacccc aacacccacc 9840
ggcacacaga ccccaaccac gacacccatc accaccacca ctacggtgac cccaacccca 9900
acacccaccg gcacacagac cccaaccacg acacccatca ccaccaccac tacggtgacc 9960
ccaaccccaa cacccaccgg cacacagacc ccaaccacga cacccatcac caccaccact 10020
acggtgaccc caaccccaac acccaccggc acacagaccc caaccacgac acccatcacc 10080
accaccacta cggtgacccc aaccccaaca cccaccggca cacagacccc aaccacgaca 10140
cccatcacca ccaccactac ggtgacccca accccaacac ccaccggcac acagacccca 10200

accacgacac ccatcaccac caccactacg gtgaccccaa ccccaacacc caccggcaca 10260
cagaccccaa ccacgacacc catcaccacc accactacgg tgaccccaac cccaacaccc 10320
accggcacac agaccccaac cacgacaccc atcaccacca ccactacggt gaccccaacc 10380
ccaacaccca ccggcacaca gaccccaacc acgacaccca tcaccaccac cactacggtg 10440
accccaaccc caacacccac cggcacacag accccaacca cgacacccat caccaccacc 10500
actacggtga ccccaacccc aacacccacc ggcacacaga ccccaaccac gacacccatc 10560
accaccacca ctacggtgac cccaacccca acacccaccg gcacacagac cccaaccacg 10620
acacccatca ccaccaccac tacggtgacc ccaaccccaa cacccaccgg cacacagacc 10680
ccaaccacga cacccatcac caccaccact acggtgaccc caaccccaac acccaccggc 10740
acacagaccc caaccacgac acccatcacc accaccacta cggtgacccc aaccccaaca 10800
cccaccggca cacagacccc aaccacgaca cccatcacca ccaccactac ggtgacccca 10860
accccaacac ccaccggcac acagacccca accacgacac ccatcaccac caccactacg 10920
gtgaccccaa ccccaacacc caccggcaca cagaccccaa ccacgacacc catcaccacc 10980
accactacgg tgaccccaac cccaacaccc accggcacac agaccccaac cacgacaccc 11040
atcaccacca ccactacggt gaccccaacc ccaacaccca ccggcacaca gaccccaacc 11100
acgacaccca tcaccaccac cactacggtg accccaaccc caacacccac cggcacacag 11160
accccaacca cgacacccat caccaccacc actacggtga ccccaacccc aacacccacc 11220
ggcacacaga ccccaaccac gacacccatc accaccacca ctacggtgac cccaacccca 11280
acacccaccg gcacacagac cccaaccacg acacccatca ccaccaccac tacggtgacc 11340
ccaaccccaa cacccaccgg cacacagacc ccaaccacga cacccatcac caccaccact 11400
acggtgaccc caaccccaac acccaccggc acacagaccc caaccacgac acccatcacc 11460
accaccacta cggtgacccc aaccccaaca cccaccggca cacagacccc aaccacgaca 11520
cccatcacca ccaccactac ggtgacccca accccaacac ccaccggcac acagacccca 11580
accacgacac ccatcaccac caccactacg gtgaccccaa ccccaacacc caccggcaca 11640
cagaccccaa ccacgacacc catcaccacc accactacgg tgaccccaac cccaacaccc 11700
accggcacac agaccccaac cacgacaccc atcaccacca ccactacggt gaccccaacc 11760
ccaacaccca ccggcacaca gaccccaacc acgacaccca tcaccaccac cactacggtg 11820
accccaaccc caacacccac cggcacacag accccaacca cgacacccat caccaccacc 11880
actacggtga ccccaacccc aacacccacc ggcacacaga ccccaaccac gacacccatc 11940
accaccacca ctacggtgac cccaacccca acacccaccg gcacacagac cccaaccacg 12000
acacccatca ccaccaccac tacggtgacc ccaaccccaa cacccaccgg cacacagacc 12060
ccaaccacga cacccatcac caccaccact acggtgaccc caaccccaac acccaccggc 12120
acacagaccc caaccacgac acccatcacc accaccacta cggtgacccc aaccccaaca 12180
cccaccggca cacagacccc aaccacgaca cccatcacca ccaccactac ggtgacccca 12240

accccaacac ccaccggcac acagacccca accacgacac ccatcaccac caccactacg 12300
gtgaccccaa ccccaacacc caccggcaca cagaccccaa ccacgacacc catcaccacc 12360
accactacgg tgaccccaac cccaacaccc accggcacac agaccccaac cacgacaccc 12420
atcaccacca ccactacggt gaccccaacc ccaacaccca ccggcacaca gaccccaacc 12480
acgacaccca tcaccaccac cactacggtg accccaaccc caacacccac cggcacacag 12540
accccaacca cgacacccat caccaccacc actacggtga ccccaacccc aacacccacc 12600
ggcacacaga ccgggccccc cacccacaca agcacagcac cgattgctga gttgaccaca 12660
tccaatcctc cgcctgagtc ctcaacccct cagacctctc ggtccacctc ttcccctctc 12720
acggagtcaa ccacccttct gagtacccta ccacctgcca ttgagatgac cagcacggcc 12780
ccaccctcca cacccacggc acccacgacc acgagcggag gccacacact gtctccaccg 12840
cccagcacca ccacgtcccc tccaggcacc cccactcgcg gtaccacgac cgggtcatct 12900
tcagccccca cccccagcac tgtgcagacg accaccacca gtgcctggac cccaacgccg 12960
accccactct ccacacccag catcatcagg accacaggcc tgaggcccta cccttcctct 13020
gtgcttatct gctgtgtcct gaacgacacc tactacgcac caggtgagga ggtgtacaac 13080
ggcacatacg gagacacctg ttatttcgtc aactgctcac tgagctgtac gttggagttc 13140
tataactggt cctgcccatc cacgccctcc ccaacaccca cgccctccaa gtcgacgccc 13200
acgccttcca agccatcgtc cacgccctcc aagccgacgc ccggcaccaa gccccccgag 13260
tgcccagact ttgatcctcc cagacaggag aacgagactt ggtggctgtg cgactgcttc 13320
atggccacgt gcaagtacaa caacacggtg gagatcgtga aggtggagtg tgagccgccg 13380
cccatgccca cctgctccaa cggcctccaa cccgtgcgcg tcgaggaccc cgacggctgc 13440
tgctggcact gggagtgcga ctgctactgc acgggctggg gcgacccgca ctatgtcacc 13500
ttcgacggac tctactacag ctaccagggc aactgcacct acgtgctggt ggaggagatc 13560
agcccctccg tggacaactt cggagtttac atcgacaact accactgcga tcccaacgac 13620
aaggtgtcct gtccccgcac cctcatcgtg cgccacgaga cccaggaggt gctgatcaag 13680
accgtgcata tgatgcccat gcaggtgcag gtgcaggtga acaggcaggc ggtggcactg 13740
ccctacaaga agtacgggct ggaggtgtac cagtctggca tcaactacgt ggtggacatc 13800
cccgagctgg gtgtcctcgt ctcctacaat ggcctgtcct tctccgtcag gctgccctac 13860
caccggtttg gcaacaacac caagggccag tgtggcacct gcaccaacac cacctccgac 13920
gactgcattc tgcccagcgg ggagatcgtc tccaactgtg aggctgcggc tgaccagtgg 13980
ctggtgaacg acccctccaa gccacactgc ccccacagca gctccacgac caagcgcccg 14040
gccgtcactg tgcccggggg cggtaaaacg accccacaca aggactgcac cccatctccc 14100
ctctgccagc tcatcaagga cagcctgttt gcccagtgcc acgcactggt gcccccgcag 14160
cactactacg atgcctgcgt gttcgacagc tgcttcatgc cgggctcgag cctggagtgc 14220
gccagtctgc aggcctacgc agccctctgt gcccagcaga acatctgcct cgactggcgg 14280
aaccacacgc atggggcctg cttggtggag tgcccatctc acagggagta ccaggcctgt 14340

ggccctgcag aagagcccac gtgcaaatcc agctcctccc agcagaacaa cacagtcctg 14400

gtggaaggct gcttctgtcc tgagggcacc atgaactacg ctcctggctt tgatgtctgc 14460

gtgaagacct gcggctgtgt gggacctgac aatgtgccca gagagtttgg ggagcacttc 14520

gagttcgact gcaagaactg tgtctgcctg gagggtggaa gtggcatcat ctgccaaccc 14580

aagaggtgca gccagaagcc cgttacccac tgcgtggaag acggcaccta cctcgccacg 14640

gaggtcaacc ctgccgacac ctgctgcaac attaccgtct gcaagtgcaa caccagcctg 14700

tgcaaagaga agccctccgt gtgcccgctg ggattcgaag tgaagagcaa gatggtgcct 14760

ggaaggtgct gtcccttcta ctggtgtgag tccaaggggg tgtgtgttca cgggaatgct 14820

gagtaccagc ccggttctcc agtttattcc tccaagtgcc aggactgcgt gtgcacggac 14880

aaggtggaca acaacaccct gctcaacgtc atcgcctgca cccacgtgcc ctgcaacacc 14940

tcctgcagcc ctggcttcga actcatggag gcccccgggg agtgctgtaa gaagtgtgaa 15000

cagacgcact gtatcatcaa acggcccgac aaccagcacg tcatcctgaa gcccggggac 15060

ttcaagagcg acccgaagaa caactgcaca ttcttcagct gcgtgaagat ccacaaccag 15120

ctcatctcgt ccgtctccaa catcacctgc cccaactttg atgccagcat ttgcatcccg 15180

ggctccatca cattcatgcc caatggatgc tgcaagacct gcacccctcg caatgagacc 15240

agggtgccct gctccaccgt ccccgtcacc acggaggttt cgtacgccgg ctgcaccaag 15300

accgtcctca tgaatcattg ctccgggtcc tgcgggacat ttgtcatgta ctcggccaag 15360

gcccaggccc tggaccacag ctgctcctgc tgcaaagagg agaaaaccag ccagcgtgag 15420

gtggtcctga gctgccccaa tggcggctcg ctgacacaca cctacaccca catcgagagc 15480

tgccagtgcc aggacaccgt ctgcgggctc cccaccggca cctcccgccg ggcccggcgc 15540

tcccctaggc atctggggag cgggtgagcg gggtgggcac agcccccttc actgccctcg 15600

acagctttac ctcccccgga ccctctgagc ctcctaagct cggcttcctc tcttcagata 15660

tttattgtct gagtctttgt tcagtccttg ctttccaata ataaactcag ggggacatgc 15720

<210> 4
<211> 1536
<212> DNA
<213> Homo sapiens

<220>
<221> misc_feature
<222> (1)..(84)
<223> >gi|20149499|ref|NM_000190.2| Homo sapiens hydroxymethylbilane
synthase (HMBS), mRNA

<400> 4
gtgacgcgag gctctgcgga gaccaggagt cagactgtag gacgacctcg ggtcccacgt 60

gtccccggta ctcgccggcc ggagcccccg gcttcccggg gccgggggac cttagcggca 120

cccacacaca gcctactttc caagcggagc catgtctggt aacggcaatg cggctgcaac 180

ggcggaagaa aacagcccaa agatgagagt gattcgcgtg ggtacccgca agagccagct 240

tgctcgcata cagacggaca gtgtggtggc aacattgaaa gcctcgtacc ctggcctgca 300

gtttgaaatc attgctatgt ccaccacagg ggacaagatt cttgatactg cactctctaa 360

gattggagag aaaagcctgt ttaccaagga gcttgaacat gccctggaga agaatgaagt 420

ggacctggtt gttcactcct tgaaggacct gcccactgtg cttcctcctg gcttcaccat 480

cggagccatc tgcaagcggg aaaaccctca tgatgctgtt gtctttcacc caaaatttgt 540

tgggaagacc ctagaaaccc tgccagagaa gagtgtggtg ggaaccagct ccctgcgaag 600

agcagcccag ctgcagagaa agttcccgca tctggagttc aggagtattc ggggaaacct 660

caacacccgg cttcggaagc tggacgagca gcaggagttc agtgccatca tcctggcaac 720

agctggcctg cagcgcatgg gctggcacaa ccgggtgggg cagatcctgc accctgagga 780

atgcatgtat gctgtgggcc agggggcctt gggcgtggaa gtgcgagcca aggaccagga 840

catcttggat ctggtgggtg tgctgcacga tcccgagact ctgcttcgct gcatcgctga 900

aagggccttc ctgaggcacc tggaaggagg ctgcagtgtg ccagtagccg tgcatacagc 960

tatgaaggat gggcaactgt acctgactgg aggagtctgg agtctagacg gctcagatag 1020

catacaagag accatgcagg ctaccatcca tgtccctgcc cagcatgaag atggccctga 1080

ggatgaccca cagttggtag gcatcactgc tcgtaacatt ccacgagggc cccagttggc 1140

tgcccagaac ttgggcatca gcctggccaa cttgttgctg agcaaaggag ccaaaaacat 1200

cctggatgtt gcacggcagc ttaacgatgc ccattaactg gtttgtgggg cacagatgcc 1260

tgggttgctg ctgtccagtg cctacatccc gggcctcagt gccccattct cactgctatc 1320

tggggagtga ttaccccggg agactgaact gcagggttca agccttccag ggatttgcct 1380

caccttgggg ccttgatgac tgccttgcct cctcagtatg tgggggcttc atctctttag 1440

agaagtccaa gcaacagcct ttgaatgtaa ccaatcctac taataaacca gttctgaagg 1500

taaaaaaaaa aaaaaaaaaa aaaaaaaaaa aaaaaa 1536

<210> 5
<211> 20
<212> DNA
<213> Artificial

<220>
<223> PCR primer

<400> 5
atgaactacg ctcctggctt 20

<210> 6
<211> 21
<212> DNA
<213> Artificial

<220>
<223> PCR primer

<400> 6
aaactctctg ggcacattgt c 21

<210>  7
<211>  21
<212>  DNA
<213>  Artificial

<220>
<223>  PCR probe

<400>  7
cgtgaagacc tgcggctgtg t                                                21


<210>  8
<211>  21
<212>  DNA
<213>  Artificial

<220>
<223>  PCR primer

<400>  8
accacagtca cgacgatcac a                                                21


<210>  9
<211>  19
<212>  DNA
<213>  Artificial

<220>
<223>  PCR primer

<400>  9
cggggttgga gttgttgct                                                   19


<210>  10
<211>  24
<212>  DNA
<213>  Artificial

<220>
<223>  PCR Probe

<400>  10
ctatgcagag ccacccaaac cctt                                             24


<210>  11
<211>  22
<212>  DNA
<213>  Artificial

<220>
<223>  PCR primer

<400>  11
cacccttcag ggtcttgaga tt                                               22


<210>  12
<211>  20
<212>  DNA
<213>  Artificial

<220>
<223>  PCR primer

<400>  12

tccgtttctg ccagtgtgtc                                                    20

<210>  13
<211>  24
<212>  DNA
<213>  Artifical

<220>
<223>  PCR primer

<400>  13
acagctgagc atgaaagctg cctt                                               24

<210>  14
<211>  22
<212>  DNA
<213>  Artifical

<220>
<223>  PCR primer

<400>  14
ctgcttcgct gcatcgctga aa                                                 22

<210>  15
<211>  22
<212>  DNA
<213>  Artificial

<220>
<223>  PCR primer

<400>  15
cagactcctc cagtcaggta ca                                                 22

<210>  16
<211>  30
<212>  DNA
<213>  Artificial

<220>
<223>  PCR probe

<400>  16
cctgaggcac ctggaaggag gctgcagtgt                                         30